# EUROPEAN PATENT SPECIFICATION

(11) **EP 2 229 433 B1**
(45) Date of publication and mention of the grant of the patent: **20.09.2017**
(21) Application number: 09700353.7
(22) Date of filing: 09.01.2009
(51) Int. Cl.: C12N 5/077, A61K 35/12, C12N 5/0775

(54) **OSTEOGENIC DIFFERENTIATION OF BONE MARROW STEM CELLS AND MESENCHYMAL STEM CELLS USING A COMBINATION OF GROWTH FACTORS**
OSTEOGENE DIFFERENZIERUNG VON KNOCHENMARKSTAMMZELLEN UND MESENCHYMALEN STAMMZELLEN UNTER VERWENDUNG EINER KOMBINATION AUS WACHSTUMSFAKTOREN
DIFFÉRENCIATION OSTÉOGÉNIQUE DE CELLULES SOUCHES DE MOELLE OSSEUSE ET DE CELLULES SOUCHES MÉSENCHYMATEUSES À L'AIDE D'UNE COMBINAISON DE FACTEURS DE CROISSANCE

(30) Priority: 11.01.2008 EP 08000458
(43) Date of publication of application: 22.09.2010
(73) Proprietor: Bone Therapeutics S.A., 6041 Gosselies (BE)
(72) Inventor: BADOER, Cindy, 1460 Ittre (BE); BASTIANELLI, Enrico, B-1640 Rhode-St-Genèse (BE); PESESSE, Xavier, B-1430 Bierghes (BE)
(74) Representative: Michalík, Andrej
(86) International application number: PCT/EP2009/050209
(87) International publication number: WO 2009/087213

(56) References cited:
- WO-A-2005/032344
- WO-A-2006/134921
- WO-A-2007/002210
- WO-A-2007/093431
- DENG ZHONG-LIANG ET AL: "Regulation of osteogenic differentiation during skeletal development." FRONTIERS IN BIOSCIENCE : A JOURNAL AND VIRTUAL LIBRARY 2008, vol. 13, 1 January 2008 (2008-01-01), pages 2001-2021, XP009101146 ISSN: 1093-4715
- MAJUMDAR M K ET AL: "Human marrow-derived mesenchymal stem cells (MSCs) express hematopoietic cytokines and support long-term hematopoiesis when differentiated toward stromal and osteogenic lineages." JOURNAL OF HEMATOTHERAPY & STEM CELL RESEARCH DEC 2000, vol. 9, no. 6, December 2000 (2000-12), pages 841-848, XP002482890 ISSN: 1525-8165
- LUU HUE H ET AL: "Distinct roles of bone morphogenetic proteins in osteogenic differentiation of mesenchymal stem cells." JOURNAL OF ORTHOPAEDIC RESEARCH : OFFICIAL PUBLICATION OF THE ORTHOPAEDIC RESEARCH SOCIETY MAY 2007, vol. 25, no. 5, May 2007 (2007-05), pages 665-677, XP002482891 ISSN: 0736-0266
- NG F. ET AL: "PDGF, TGF-b and FGF signaling is important for differentiation and growth of mesenchymal stem cells (MSCs): transcriptional profiling can identify markers and signaling pathways important in differentiation of MSC into adipogenic, chondrogenic and ostoegenic lineages." BLOOD, vol. 1, 10 March 2008 (2008-03-10), XP002482908
- LOCKLIN R M ET AL: "Effects of TGFbeta and bFGF on the differentiation of human bone marrow stromal fibroblasts.", CELL BIOLOGY INTERNATIONAL 1999, vol. 23, no. 3, 1999, pages 185-194, ISSN: 1065-6995

## Description

### Field of the Invention

The invention relates to methods for osteogenic differentiation of bone marrow stem cells (BMSC) and mesenchymal stem cells (MSC), to cells and cell populations obtainable by said methods and to uses thereof particularly in the field of bone therapy.

### Background to the Invention

Transplantation of stem cells capable of undergoing osteogenic differentiation or of cells that are committed towards osteogenic differentiation is a promising avenue for the treatment of bone-related diseases, in particular when the treatment requires production of new bone.

Bone marrow stem cells (BMSC) and mesenchymal stem cells (MSC) have been used previously to treat bone disorders (Gangji et al., 2005 Expert Opin Biol Ther 5: 437-42). However, although such relatively undifferentiated stem cells can be transplanted, they are not committed to an osteogenic lineage and therefore a considerable proportion of so-transplanted stem cells may not eventually contribute to the formation of the desired bone tissue.

Moreover, the quantity of such stem cells obtainable from bone marrow or other source tissues is frequently unsatisfactory and may be even further reduced in various circumstances, such as due to glucocorticoid use or alcohol abuse or because of genetic causes.

WO 2007/093431 disclosed a method that achieved an adequate extent of *in vitro* expansion of isolated BMSC or MSC and yielded cells that already displayed osteoblastic phenotype. In said method, human BMSC or MSC were cultured in the presence of serum or plasma and basic fibroblast growth factor (FGF). WO 2007/093431 does not teach using the specific combination of FGF and TGF growth factors for obtaining osteoblasts and does not suggest any advantages thereof.

There exists a need for further simple and reliable methods for producing useful osteoprogenitors, osteoblasts or osteoblastic phenotype cells from BMSC and MSC. Such methods are particularly desired where they achieve a greater degree of cell expansion and/or produce cells with additional advantageous properties, such as for example, cells causing less tissue rejection in allogeneic subjects.

It is an object of the invention to provide such methods and osteoblast phenotype cells obtainable thereby.

### Summary of the Invention

The inventors realised that the expansion potential of adult stem cells, more specifically of bone marrow stem cells (BMSC) and mesenchymal stem cells (MSC), can be considerably augmented upon osteogenic differentiation when said cells are cultured in the presence of serum or plasma, fibroblast growth factor (FGF) and transforming growth factor beta (TGFB), whereby increased quantities of useful osteoprogenitor or osteoblast phenotype cells and cell populations can be achieved.

The present invention provides subject-matter as set forth in any one and all of the appended claims 1 to 16.

The subject-matter provided by the invention thus specifically belongs to the disclosure, description and teaching of the present specification.

Hence, in an aspect the invention provides a method for obtaining osteoprogenitors or osteoblasts from adult human bone marrow stem cells (BMSC) or adult human mesenchymal stem cells (MSC) *in vitro* or *ex vivo,* comprising culturing said BMSC or MSC in a medium including human plasma or serum, fibroblast growth factor (FGF) and transforming growth factor beta (TGFB).

The specification further describes a method for obtaining osteoprogenitors, osteoblasts or osteoblast phenotype cells from human bone marrow stem cells (BMSC) or human mesenchymal stem cells (MSC) *in vitro* or *ex vivo,* comprising contacting said BMSC or MSC with human plasma or serum, fibroblast growth factor (FGF) or a biologically active variant or derivative thereof, and transforming growth factor beta (TGFB) or a biologically active variant or derivative thereof.

The present methods can differentiate a substantial fraction, e.g. a majority, of treated stem cells toward osteoprogenitor or osteoblast phenotypes. Hence, the methods of the invention can be employed for obtaining osteoprogenitors, osteoblasts or osteoblast phenotype cells *per se.*

It shall however be appreciated that the methods of the invention may generally produce cell populations comprising a substantial portion, e.g. a majority, of osteoprogenitors, osteoblasts or osteoblast phenotype cells. Such cell populations may optionally include further cell types.

Accordingly, the specification contemplates a method for obtaining a cell population comprising osteoprogenitors, osteoblasts or osteoblast phenotype cells from human BMSC or human MSC *in vitro* or *ex vivo,* comprising contacting said BMSC or MSC with human plasma or serum, FGF or a biologically active variant or derivative thereof, and TGFB or a biologically active variant or derivative thereof.

The present methods allow to generate high numbers of osteogenic cells for the purposes of transplantation. This permits to further reduce the amount of tissue which needs to be drawn from a subject to obtain the starting BMSC or MSC cells. This as well permits to obtain adequate numbers of osteogenic cells from subjects with diminished counts of BMSC or MSC cells. Also, the methods allow for shortening the time when the differentiated cells can be transplanted into a patient, thus resulting in faster therapy.

In an embodiment, the above methods may comprise steps:
(a) recovering cells from a biological sample of a human subject comprising BMSC or MSC;
(b) optionally, isolating mono-nucleated cells from the cells recovered in (a);
(c) adding cells of (a) or (b) to a medium comprising human plasma or serum, FGF and TGFB, and culturing the cell-medium mixture, such as to allow for adherence of cells to a substrate surface;
(d) removing non-adherent matter and further culturing adherent cells in the medium as defined in (c), such as to allow for obtaining osteoprogenitors, osteoblasts or osteoblast-like cells, or a cell population comprising such.

In an embodiment of any of the above methods, BMSC or MSC cells may be exposed to FGF and TGFB concurrently, i.e. simultaneously.

In further embodiments of any of the above methods, the human plasma or serum may be autologous to the BMSC or MSC cells, or it may be allogeneic (homologous) to the BMSC or MSC cells.

In a further embodiment of any of the above methods, no non-human animal material (such as, e.g., serum components) is used in the culture of the BMSC or MSC cells. This decreases the risk of xenogenic rejection of the cultured cells in humans as well as reduces the risk of contamination of patients with pathogens.

Additional preferred embodiments of the above methods concern further features, such as without limitation, incubation times, passages, component quantities, etc., which alone or in combination further underlie the provision of the present cells and cell populations.

The inventors further surprisingly realised that the expression of HLA-DR major histocompatibility complex in osteoblast phenotype cells obtained using the present methods is significantly lower than HLA-DR expression observed when applying the method of WO 2007/093431. Such lower HLA-DR expression can enhance the immunoprivileged character of osteoblast phenotype cells as obtained herein, thereby reducing the risk of rejection of such cells and allowing broader application thereof also in allogeneic patients.

The invention thus also provides osteoblast phenotype cells displaying novel and advantageous properties over prior art. Accordingly, in aspects the invention provides a cell population comprising human osteoprogenitors or osteoblasts, characterised in that said osteoprogenitors or osteoblasts (1) comprise expression of CD90, CD105, CD73, CD63, CD166, alkaline phosphatase (ALP), more specifically ALP of the bone-liver-kidney type, (2) do not express CD45, CD14, CD19, and (3) less than 15% of the cells express HLA-DR.

The specification further describes osteoprogenitors, osteoblasts or osteoblast phenotype cells, as well as cell populations comprising such, that are obtainable or directly obtained using the methods of the invention.

The inventors also analysed the herein achieved osteoblastic cells to define a new cell type and cell populations comprising such, that may offer particular superiority in therapy, especially in bone transplantation therapy.

Hence, the specification provides human osteoprogenitors, osteoblasts or osteoblast phenotype cells, as well as cell populations comprising such, characterised in that said cells (1) comprise expression of CD90, CD105, CD73, CD63, CD166 and alkaline phosphatase (ALP), more specifically ALP of the bone-liver-kidney type, (2) do not express CD45, CD14, CD19, and (3) less than 25% of the cells, preferably less than 20% of the cells and even more preferably less than 15% of the cells.

In certain embodiments, less than 10% of the cells or less than 7% of the cells express HLA-DR (i.e., a sizeable proportion of the cells do not express HLA-DR).

As mentioned, the invention also contemplates cell populations comprising the osteoprogenitor, osteoblast or osteoblast phenotype cells as defined herein. An exemplary cell population may comprise at least 10%, preferably at least 30%, more preferably at least 50%, e.g., at least 60%, yet more preferably at least 70%, e.g., at least 80%, and even more preferably at least 90%, e.g., at least 95% of osteoprogenitor, osteoblast or osteoblast phenotype cells as defined herein. For example, the cell population may comprise less than 50%, preferably less than 40%, even more preferably less than 30%, yet more preferably less than 20% and still more preferably less than 10%, e.g., less than 7%, less than 5% or less than 2% of cell types other than the osteoprogenitor, osteoblast or osteoblast phenotype cells as defined herein.

Related aspects concern therapeutic uses of osteoprogenitors, osteoblasts or osteoblast phenotype cells, or of cell populations comprising such, as taught by the invention, in bone-related disorders and corresponding pharmaceutical formulations comprising said cells or cell populations.

These and other features of the invention are further explained here below and in the appended claims, as well as illustrated by non-limiting examples.

### Brief Description of the Figures

**Figure 1** shows box-whisker plot comparison of osteoblast primary culture yield in medium containing plasma and 10 ng/ml TGFB-1 or containing plasma and both 10ng/ml FGF2 and 10 ng/ml TGFb-1 (n=4).
**Figure 2** shows comparison of osteoblast culture yield in medium supplemented *vs*. not supplemented with 10 ng/ml TGFb-1. (A) primary culture, (B) secondary culture, (C) global.
**Figure 3** shows mineralization by cells cultured in presence of (A) FGF2 only or (B) FGF2 and TGFb-1, assayed by matrix alizarin red staining.
**Figure 4** illustrates immunosuppressive effect of cells cultured in TGFb-1/FGF2 on PBMC stimulated by PHA.

### Detailed Description of the Invention

As used herein, the singular forms "a", "an", and "the" include both singular and plural referents unless the context clearly dictates otherwise. By way of example, "a cell" refers to one or more than one cell.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within that range, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety. In particular, the teachings of all documents herein specifically referred to are incorporated by reference.

Unless otherwise defined, all terms used in disclosing the invention, including technical and scientific terms, have the meaning as commonly understood by one of ordinary skill in the art to which this invention belongs.

### 1. Methods of invention

As detailed in the Summary section, in an aspect, the invention relates to a method for obtaining osteoprogenitors, osteoblasts or osteoblast phenotype cells, as well as for obtaining cell populations comprising osteoprogenitors, osteoblasts or osteoblast phenotype cells, from human bone marrow stem cells *in vitro* or *ex vivo.*

As used herein, the recitation osteoprogenitors, osteoblasts or osteoblast phenotype cells generally encompass cells which can contribute to, or are capable of developing to cells which can contribute to, the formation of bone material or bone matrix. In particular, the methods of the invention result in cells and cell populations which, as experimentally substantiated by the inventors, are useful for restoring bone formation in therapeutic settings. Consequently, the recitation osteoprogenitors, osteoblasts or osteoblast phenotype cells should be construed as wishing to encompass any such useful cells of the osteogenic lineage resulting from the methods of the invention.

Bone marrow is the soft tissue occupying medullar cavities of long bones, some haversian canals, and spaces between trabeculae of cancellous or spongy bone. The term particularly but without limitation encompasses red bone marrow, which is found in all bones in early life and in restricted locations in adulthood (e.g., in the spongy bone), as well as yellow bone marrow.

As a whole, bone marrow is a complex tissue comprised *inter alia* of haematopoietic stem cells, red and white blood cells and their precursors, mesenchymal stem cells (MSC), stromal cells and their precursors, and a group of cells including fibroblasts, reticulocytes, adipocytes, and cells which form a connective tissue network called "stroma".

Bone marrow cells contribute to many diverse tissues after systemic transplantation in both mice and humans. This capacity may reflect the activities of multiple stem cells present in bone marrow, such as, e.g., haematopoietic stem cells, mesenchymal stem cells and/or marrow multipotent stem cell.

The term "bone marrow stem cell" or "BMSC" as used herein thus refers to any adult stem cell present in bone marrow, and particularly present in or (partly) isolated from a sample of bone marrow. A sample of bone marrow (BMSC) may be obtained, e.g., from iliac crest, femora, tibiae, spine, rib or other medullar spaces of a subject. The term BMSC also encompasses the progeny of BMSC, e.g., progeny obtained by *in vitro* or *ex vivo* propagation of BMSC obtained from a sample of a subject.

The term "isolating" with reference to a particular component denotes separating that component from at least one other component of a composition from which the former component is being isolated. The term "isolated" as used herein in relation to any cell population also implies that such cell population does not form part of an animal or human body.

The term "stem cell" refers generally to an unspecialised or relatively less specialised and proliferation-competent cell, which is capable of self-renewal, i.e., can proliferate without differentiation, and which or the progeny of which can give rise to at least one relatively more specialised cell type. The term encompasses stem cells capable of substantially unlimited self-renewal, i.e., wherein the progeny of a stem cell or at least part thereof substantially retains the unspecialised or relatively less specialised phenotype, the differentiation potential, and the proliferation capacity of the mother stem cell, as well as stem cells which display limited self-renewal, i.e., wherein the capacity of the progeny or part thereof for further proliferation and/or differentiation is demonstrably reduced compared to the mother cell. By means of example and not limitation, a stem cell may give rise to descendants that can differentiate along one or more lineages to produce increasingly relatively more specialised cells, wherein such descendants and/or increasingly relatively more specialised cells may themselves be stem cells as defined herein, or even to produce terminally differentiated cells, i.e., fully specialised cells, which may be post-mitotic.

The term "adult stem cell" as used herein refers to a stem cell present in or obtained from (such as isolated from) an organism at the foetal stage or after birth.

Preferable bone marrow stem cells as defined herein have the potential of generating cells of at least the osteogenic (bone) lineage, such as, e.g., osteogenic cells and/or osteoprogenitors and/or pre-osteoblasts and/or osteoblasts and/or osteocytes, etc.

Preferably, at least some bone marrow stem cells as defined herein may also have the potential to generate further cells comprised in the cell populations resulting from the methods of the invention, such as, e.g., cells of endothelial lineage, for example endothelial progenitor cells and/or endothelial cells.

An exemplary, but non-limiting, type of BMSC having the potential of generating cells of at least the osteogenic lineage are mesenchymal stem cells. The term "mesenchymal stem cell" or "MSC" as used herein refers to an adult, mesoderm-derived stem cell that is capable of generating cells of mesenchymal lineages, typically of two or more mesenchymal lineages, e.g., osteocytic (bone), chondrocytic (cartilage), myocytic (muscle), tendonocytic (tendon), fibroblastic (connective tissue), adipocytic (fat) and stromogenic (marrow stroma) lineage. MSC may be isolated from, e.g., bone marrow, blood, umbilical cord, placenta, foetal yolk sac, skin (dermis), specifically foetal and adolescent skin, periosteum and adipose tissue. Human MSC, their isolation, *in vitro* expansion, and differentiation, have been described in, e.g., US Pat. No. 5,486,359; US Pat. No. 5,811,094; US Pat. No. 5,736,396; US Pat. No. 5,837,539; or US Pat. No. 5,827,740. Any MSC described in the art and isolated by any method described in the art may be suitable in the present invention, provided such MSC are capable of generating cells of at least the osteocytic (bone) lineage.

The term MSC also encompasses the progeny of MSC, e.g., progeny obtained by *in vitro* or *ex vivo* propagation of MSC obtained from a biological sample of an animal or human subject.

Potentially, but without limitation, at least some MSC might also be able to generate further cells comprised in the cell populations resulting from the methods of the invention.

As shown in the examples, the present method of entails selecting those BMSC cells which under the specified culture conditions adhere to a substrate surface. It is known in the art that MSC can be isolated from bone marrow (or other sources) by selecting those (mononuclear) cells which can adhere to a substrate surface, e.g., plastic surface. Therefore, without being limited to any hypothesis, the present inventors speculate that in the present method, MSC may at least partly contribute to obtaining of osteoblasts or osteoblast phenotype cells from BMSC.

Therefore, the present specification also contemplates a method for obtaining osteoprogenitors, osteoblasts or osteoblast phenotype cells from human mesenchymal stem cells (MSC) *in vitro* or *ex vivo* comprising contacting said MSC cells with human plasma or serum, FGF or a biologically active variant or derivative thereof, and TGFB or a biologically active variant or derivative thereof.

MSC may be comprised in a biological sample, e.g., in a sample comprising BMSC, or may be at least partly isolated there from as known in the art. Moreover, MSC may be at least partly isolated from bone marrow or from any suitable sources comprising MSC other than bone marrow, e.g., blood, umbilical cord, placenta, foetal yolk sac, skin (dermis), specifically foetal and adolescent skin, periosteum and adipose tissue.

BMSC cells intend to encompass any and all subtypes thereof, such as without limitation, "rapidly self-renewing cells" RS-1 or RS-2 as described in Colter et al. 2000 (PNAS 97(7): 3213-8); "side population" (SP) cells as described by Goodell et al. 1997 (Nat Med 3(12): 1337-45); osteogenic precursor (OP) cells which are initially identified by their low density (e.g., upon density gradient centrifugation), non-adherent nature and low-level of expression of osteogenic markers (as described by Long et al. 1995. J Clin Invest. 95(2): 881-7; US 5,972,703); primitive precursor cells which can generate cells of both the haematopoietic and non-haematopoietic lineages as described by Krause et al. 2001 (Cell 105: 369-377) and Dominici et al. 2004. (PNAS 101 (32): 11761-6); and others.

It is to be understood that, given the complexity of bone marrow stem cell populations, the present invention should not be seen as limited to one or more particular BMSC types. Rather, in the present method, one or more BMSC cell types, e.g., as described above, may contribute, perhaps to different extent, to obtaining osteoprogenitors, osteoblasts or osteoblast phenotype cells, or to obtaining cell populations comprising such. On the other hand, it is to be understood that the present method may also employ a particular BMSC population, e.g., MSC, at least partly isolated from other BMSC populations.

The term *"in vitro"* generally denotes outside, or external to, animal or human body. The term *"ex vivo"* typically refers to tissues or cells removed from an animal or human body and maintained or propagated outside the body, e.g., in a culture vessel. The term *"in vitro"* as used herein should be understood to include *"ex vivo".*

In an embodiment, BMSC or MSC are obtained from a biological sample of a subject.

The term "subject" as used herein refers to an animal, more particularly to non-human mammals and human organism. Non-human animal subjects may also include prenatal forms of animals, such as, e.g., embryos or foetuses. Human subjects may also include foetuses, but by preference not embryos. Human subjects are preferred.

The term "biological sample" or "sample" as used herein generally refers to a sample obtained from a biological source, e.g., from an organism, organ, tissue or cell culture, etc. A biological sample of an animal or human subject refers to a sample removed from an animal or human subject and comprising cells thereof. The biological sample of an animal or human subject may comprise one or more tissue types and cells of one or more tissue types. Methods of obtaining biological samples of an animal or human subject are well known in the art, e.g., tissue biopsy or drawing blood.

A useful biological sample of a subject comprises BMSC or MSC thereof. Such sample may be typically obtained from bone marrow, e.g., from iliac crest, femora, tibiae, spine, rib or other medullar spaces of a subject. Another useful biological sample comprising MSC may be derived, e.g., from, blood, umbilical cord, placenta, foetal yolk sac, skin (dermis), specifically foetal and adolescent skin, periosteum, or adipose tissue of a subject.

In an embodiment, BMSC or MSC may be obtained from a healthy subject, which may help to ensure the functionality of osteoblasts or osteoblast phenotype cells obtained from said BMSC or MSC.

In another embodiment, BMSC or MSC may be obtained from a subject who is at risk for or has a bone-related disorder, and who can thus particularly benefit from administration of osteoblasts or osteoblast phenotype cells obtained from said BMSC or MSC according to the methods of the invention.

The term "bone-related disorder" as used herein refers to any type of bone disease, the treatment of which may benefit from the administration of osteogenic lineage cells, e.g., osteoprogenitors, osteoblasts or osteoblast phenotype cells to a subject having the disorder. In particular, such disorders may be characterised, e.g., by decreased bone formation or excessive bone resorption, by decreased number, viability or function of osteoblasts or osteocytes present in the bone, decreased bone mass in a subject, thinning of bone, compromised bone strength or elasticity, etc.

By way of example, but not limitation, bone-related disorders which can benefit from administration of osteoblasts or osteoblast phenotype cells as described herein may include local or systemic disorders, such as, any type of osteoporosis or osteopenia, e.g., primary, postmenopausal, senile, corticoid-induced, any secondary, mono- or multisite osteonecrosis, any type of fracture, e.g., non-union, mal-union, delayed union fractures or compression, conditions requiring bone fusion (e.g., spinal fusions and rebuilding), maxillo-facial fractures, bone reconstruction, e.g., after traumatic injury or cancer surgery, cranio-facial bone reconstruction, osteogenesis imperfecta, osteolytic bone cancer, Paget's Disease, endocrinological disorders, hypophsophatemia, hypocalcemia, renal osteodystrophy, osteomalacia, adynamic bone disease, rheumatoid arthritis, hyperparathyroidism, primary hyperparathyroidism, secondary hyperparathyroidism, periodontal disease, Gorham-Stout disease and McCune-Albright syndrome.

In the present methods, BMSC or MSC are contacted with human plasma or serum, FGF or a biologically active variant or derivative thereof, and TGFB or a biologically active variant or derivative thereof.

The term "contacting" as used herein means bringing together, either directly or indirectly, one or more molecules, components or materials with another, thereby facilitating interactions there between. Typically, a growth factor or a biologically active variant or derivative thereof, and human plasma or serum, may be contacted with BMSC by means of their inclusion in the media, in which the BMSC are cultured.

A skilled person appreciates that human plasma and serum are complex biological compositions, which may comprise one or more growth factors, cytokines or hormones. Hence, it is intended that the recited growth factors FGF and TGFB or their respective biologically active variants or derivatives are provided in addition to, i.e., exogenously to or in supplement to, the plasma or serum.

In a further embodiment, BMSC or MSC may be - in addition to FGF and TGFB - contacted with one or more additional, exogenously added growth factors other than FGF and TFGB. The term "growth factor" as used herein refers to a biologically active substance which influences proliferation, growth, differentiation, survival and/or migration of various cell types, and may effect developmental, morphological and functional changes in an organism, either alone or when modulated by other substances. A growth factor may typically act by binding, as a ligand, to a receptor (e.g., surface or intracellular receptor) present in cells responsive to the growth factor. A growth factor herein may be particularly a proteinaceous entity comprising one or more polypeptide chains.

In a preferred embodiment, any one or more or all growth factors used in the present method (general reference to a growth factor as used herein particularly encompasses the FGF and TGFB growth factors, as well as the optional one or more further growth factors) is human growth factor. As used herein, the term "human growth factor" refers to a growth factor substantially the same as a naturally occurring human growth factor. For example, where the growth factor is a proteinaceous entity, the constituent peptide(s) or polypeptide(s) thereof may have primary amino acid sequence identical to a naturally occurring human growth factor. The use of human growth factors in the present method is preferred, as such growth factors are expected to elicit a desirable effect on human cellular function.

The term "naturally occurring" is used to describe an object or entity that can be found in nature as distinct from being artificially produced by man. For example, a polypeptide sequence present in an organism, which can be isolated from a source in nature and which has not been intentionally modified by man in the laboratory, is naturally occurring. When referring to a particular entity, e.g., to a polypeptide or protein, the term encompasses all forms and variants thereof which occur in nature, e.g., due to a normal variation between species and individuals. For example, when referring to a proteinaceous growth factor, the term "naturally occurring" encompasses growth factors having differences in the primary sequence of their constituent peptide(s) or polypeptide(s) due to genetic divergence between species and normal allelic variation between individuals.

The term "FGF" as used herein encompasses any and all members of the fibroblast growth factor (FGF) family of growth factors. In particular, the FGF used in the present methods may be chosen from FGF-1, FGF-2 and FGF-3, more preferably it is FGF-2.

Acidic fibroblast growth factor (FGF-1) is also commonly known as heparin-binding growth factor 1 (HBGF-1), aFGF, beta-endothelial cell growth factor, or ECGF-beta. Exemplary human FGF-1 includes, without limitation, FGF-1 having primary amino acid sequence as annotated under Uniprot/Swissprot (http://www.expasy.org/) accession number P05230. A skilled person can appreciate that said sequence is of a precursor FGF-1 and may include parts which are processed away from mature FGF-1. Exemplary human FGF-1 has been also described *inter alia* by Jaye et al. 1986 (Science 233: 541-545) and Harper et al. 1986 (Biochemistry 25: 4097-4103).

Basic fibroblast growth factor is also commonly known as FGF-b, FGF-2, BFGF, HBGH-2, prostatropin, or heparin-binding growth factor 2 precursor (HBGF-2). Exemplary human FGF-2 includes, without limitation, FGF-2 having primary amino acid sequence as annotated under Uniprot/Swissprot accession number P09038. A skilled person can appreciate that said sequence is of a precursor FGF-2 and may include parts which are processed away from mature FGF-2. Exemplary human FGF-2 has been also described *inter alia* by Abraham et al. 1986 (EMBO J 5: 2523-8) and Kurokawa et al. 1987 (FEBS Lett 213: 189-94).

Fibroblast growth factor 3 (FGF-3) is also commonly known as INT-2 proto-oncogene protein, or HBGF-3. Exemplary human FGF-3 includes, without limitation, FGF-3 having primary amino acid sequence as annotated under Uniprot/Swissprot accession number P11487. A skilled person can appreciate that said sequence is of a precursor FGF-3 and may include parts which are processed away from mature FGF-3. Exemplary human FGF-3 has been also described *inter alia* by Brooks et al. 1989 (Oncogene 4: 429-436).

The terms "TGF beta" or "TGFB" as used herein encompasses any and all members of the transforming growth factor beta (TGFB) family of growth factors. In particular, the TGFB used in the present methods may be chosen from TGFB-1, TGFB-2 and TGFB-3, more preferably it is TGFB-1.

Transforming growth factor beta-1 is also known as TGFB-1 and TGF-beta-1. Exemplary human TGFB-1 includes, without limitation, TGFB-1 having primary amino acid sequence as annotated under Uniprot/Swissprot accession number P01137. A skilled person can appreciate that said sequence is of a precursor TGFB-1 and may include parts which are processed away from mature TGFB-1. Exemplary human TGFB-1 has been also described *inter alia* by Derynck et al. 1985 (Nature 316: 701-5).

Transforming growth factor beta-2 is also known as TGFB-2, glioblastoma-derived T-cell suppressor factor, G-TSF, BSC-1 cell growth inhibitor, polyergin, or cetermin. Exemplary human TGFB-2 includes, without limitation, TGFB-2 having primary amino acid sequence as annotated under Uniprot/Swissprot accession number P61812. A skilled person can appreciate that said sequence is of a precursor TGFB-2 and may include parts which are processed away from mature TGFB-2. Exemplary human TGFB-2 has been also described *inter alia* by Webb et al. 1988 (DNA 7: 493-497).

Transforming growth factor beta-3 is also known as TGFB-3. Exemplary human TGFB-3 includes, without limitation, TGFB-3 having primary amino acid sequence as annotated under Uniprot/Swissprot accession number P10600. A skilled person can appreciate that said sequence is of a precursor TGFB-3 and may include parts which are processed away from mature TGFB-3. Exemplary human TGFB-3 has been also described *inter alia* by ten Dijke et al. 1988 (PNAS 85: 4715-4719) and Derynck et al. 1988 (EMBO J. 7: 3737-3743).

The present method may employ biologically active variants or derivatives of any one or more or all of the respective growth factors. In the methods as described herein, "biologically active" variants or derivatives of the growth factor achieve at least about the same degree of obtaining osteoblasts or osteoblast phenotype cells from BMSC as the respective growth factor, when other conditions are substantially the same.

Where a growth factor exerts its effects by binding to its cognate receptor, biologically active variants or derivatives of the said growth factor may display affinity and/or specificity for binding to that cognate receptor, which is at least about as high as the affinity and/or specificity of the growth factor for binding thereto. For example, the said biologically active variants or derivatives may have affinity and/or specificity for binding to the cognate receptor which is at least 80%, e.g., at least 85%, preferably at least 90%, e.g., at least 90%, or even 100% or more of the affinity and/or specificity of the respective growth factor for binding to that receptor. The above parameters of the binding may be readily determined by a skilled person using *in vitro* or cellular assays which are known *per se.*

Where the activity of a given growth factor can be readily measured in an established assay, e.g., an *in vitro* or cellular assay (such as, for example, measurement of mitogenic activity in cell culture), biologically active variants or derivatives of the said growth factor may display activity in such assays, which is at least about as high as the activity of the growth factor. For example, the said biologically active variants or derivatives may show activity which is at least 80%, e.g., at least 85%, preferably at least 90%, e.g., at least 90%, or even 100% or more of the activity of the respective growth factor.

A "variant" of a polypeptide has an amino acid sequence which is substantially identical (i.e., largely but not wholly identical) to the amino acid sequence of the polypeptide. Herein, "substantially identical" refers to at least 85% identical, at least 90% identical, preferably at least 95% identical, e.g., least 99% identical. Sequence differences may result from insertion (addition), deletion and/or substitution of one of more amino acids.

Sequence identity between two polypeptides can be determined by optimally aligning (optimal alignment of two protein sequences is the alignment that maximises the sum of pair-scores less any penalty for introduced gaps; and may be preferably conducted by computerised implementations of algorithms, such as "Gap", using the algorithm of Needleman and Wunsch 1970 (J Mol Biol 48: 443-453), or "Bestfit", using the algorithm of Smith and Waterman 1981 (J Mol Biol 147: 195-197), as available in, e.g., the GCG^{™} v. 11.1.2 package from Accelrys) the amino acid sequences of the polypeptides and scoring, on one hand, the number of positions in the alignment at which the polypeptides contain the same amino acid residue and, on the other hand, the number of positions in the alignment at which the two polypeptides differ in their sequence. The two polypeptides differ in their sequence at a given position in the alignment when the polypeptides contain different amino acid residues at that position (amino acid substitution), or when one of the polypeptides contains an amino acid residue at that position while the other one does not or vice versa (amino acid insertion or deletion). Sequence identity is calculated as the proportion (percentage) of positions in the alignment at which the polypeptides contain the same amino acid residue versus the total number of positions in the alignment. Further suitable algorithms for performing sequence alignments and determination of sequence identity include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250).

At least some of the differences between the amino acid sequences of a variant and of the respective polypeptide with which the variant is substantially identical, can involve amino acid substitutions. Preferably, at least 85%, e.g., at least 90%, more preferably at least 95%, e.g., 100% of the said differences can be amino acid substitutions. Preferably, the said amino acid substitutions may be conservative. The term "conservative substitution" as used herein denotes that one amino acid residue has been replaced by another, biologically similar amino acid residue. Non-limiting examples of conservative substitutions include the substitution of one hydrophobic amino acid residue, such as isoleucine, valine, leucine or methionine for another, or the substitution of one polar residue for another, such as between arginine and lysine, between glutamic and aspartic acids or between glutamine and asparagine, and the like.

The "variant" of polypeptide, as used herein, also specifically includes polypeptides having a certain degree of similarity to said polypeptide. Preferably, such variants can be at least 90% similar, e.g., preferably at least 91% similar, e.g., at least 92% similar, 93% similar, more preferably at least 94% similar, e.g., 95% similar, even more preferably at least 96% similar, e.g., at least 97% similar, yet more preferably at least 98% similar, e.g., at least 99% similar.

Sequence similarity between two polypeptides can be determined by optimally aligning (see above) the amino acid sequences of the polypeptides and scoring, on one hand, the number of positions in the alignment at which the polypeptides contain the same or similar (i.e., conservatively substituted) amino acid residue and, on the other hand, the number of positions in the alignment at which the two polypeptides otherwise differ in their sequence.

The two polypeptides otherwise differ in their sequence at a given position in the alignment when the polypeptides contain non-conservative amino acid residues at that position, or when one of the polypeptides contains an amino acid residue at that position while the other one does not or vice versa (amino acid insertion or deletion). Sequence similarity is calculated as the proportion (percentage) of positions in the alignment at which the polypeptides contain the same or similar amino acid residue versus the total number of positions in the alignment.

A variant growth factor may be comprised of one or more peptide(s) or polypeptide(s), at least one of which is a variant as defined above of the respective constituent peptide or polypeptide of the growth factor.

A "derivative" of a polypeptide may be derivatised by chemical alteration of one or mode amino acid residues and/or addition of one or more moieties at one or more amino acid residues, e.g., by glycosylation, phosphorylation, acylation, acetylation, sulphation, lipidation, alkylation, etc. Typically, less than 50%, e.g., less than 40%, preferably less than 30%, e.g., less than 20%, more preferably less than 15%, e.g., less than 10% or less than 5%, e.g., less than 4%, 3%, 2% or 1% of amino acids in a derivative polypeptide may be so derivatised. A derivative proteinaceous growth factor may be comprised of one or more peptide(s) or polypeptide(s), at least one of which may be derivatised on at least one amino acid residue.

In a preferred embodiment, the growth factor or a biologically active variant or derivative thereof may be recombinant, i.e., produced by a host organism through the expression of a recombinant nucleic acid molecule, which has been introduced into the host organism (e.g., bacteria such as without limitation *E. coli, S. tymphimurium, Serratia marcescens, Bacillus subtilis*; yeast such as for example *S. cerevisiae* and *Pichia pastoris*; cultured plant cells such as *inter alia Arabidopsis thaliana* and *Nicotiana tobaccum* cells; animal cells such as mammalian and insect cells; or multi-cellular organisms such as plants or animals) or an ancestor thereof, and which comprises a sequence encoding the said polypeptide. The use of recombinantly expressed growth factors or biologically active variants or derivatives thereof lowers the risk of transmission of pathogenic agents. Recombinant production of TGFB-1 has been described *inter alia* by Bourdrel et al. 1993 (Protein Expr Purif 4: 130-40). Recombinant growth factors are also commonly commercially available (e.g., from Sigma, Biological Industries, R&D Systems, Peprotech, etc.).

The term "plasma" is as conventionally defined. Plasma is usually obtained from a sample of whole blood, provided or contacted with an anticoagulant, (e.g., heparin, citrate, oxalate or EDTA). Subsequently, cellular components of the blood sample are separated from the liquid component (plasma) by an appropriate technique, typically by centrifugation. The term "plasma" therefore refers to a composition which does not form part of a human or animal body.

The term "serum" is as conventionally defined. Serum can be usually obtained from a sample of whole blood by first allowing clotting to take place in the sample and subsequently separating the so formed clot and cellular components of the blood sample from the liquid component (serum) by an appropriate technique, typically by centrifugation. Clotting can be facilitated by an inert catalyst, e.g., glass beads or powder. Alternatively, serum can be obtained from plasma by removing the anticoagulant and fibrin. The term "serum" hence refers to a composition which does not form part of a human or animal body.

The isolated plasma or serum can be used directly in the method of the present invention. They can also be appropriately stored for later use (e.g., for shorter time periods, e.g., up to about 1-2 weeks, at a temperature above the respective freezing points of plasma or serum, but below ambient temperature, this temperature will usually be about 4°C to 5°C; or for longer times by freeze storage, usually at between about -70 °C and about -80 °C).

The isolated plasma or serum can be heat inactivated as known in the art, particularly to remove the complement. Where the present method employs plasma or serum autologous to the cells cultured in the presence thereof, it may be unnecessary to heat inactivate the plasma or serum. Where the plasma or serum is at least partly allogeneic to the cultured cells, it may be advantageous to heat inactivate the plasma or serum.

Optionally, the plasma or serum may also be sterilised prior to storage or use, using conventional microbiological filters, preferably with pore size of 0.2µm or smaller.

In an embodiment, the present method may employ human plasma or serum which is autologous to human BMSC or MSC contacted therewith. The term "autologous" with reference to plasma or serum denotes that the plasma or serum is obtained from the same subject as are BMSC or MSC to be contacted with the said plasma or serum.

In another embodiment, the method may employ human plasma or serum which is "homologous" or "allogeneic" to human BMSC or MSC contacted therewith, i.e., obtained from one or more (pooled) human subjects other than the subject from which the BMSC or MSC are obtained.

In a further embodiment, the method may employ a mixture of autologous and homologous (allogeneic) plasma or sera as defined above.

As noted, the present methods may commonly comprise steps:
(a) recovering cells from a biological sample of a human subject comprising BMSC or MSC;
(b) optionally, isolating mono-nucleated cells from the cells recovered in (a);
(c) adding cells of (a) or (b) to a medium comprising human plasma or serum, FGF and TGFB, and culturing the cell-medium mixture, such as to allow for adherence of cells to a substrate surface;
(d) removing non-adherent matter and further culturing adherent cells in the medium as defined in (c), such as to allow for obtaining osteoprogenitors, osteoblasts or osteoblast-like cells, or a cell population comprising such.

Step (b) may be performed using conventional methods such as, e.g., density gradient centrifugation.

The cell suspension from steps (a) or (b) is cultured in the presence of a medium, commonly a liquid cell culture medium. Typically, the medium will comprise a basal medium formulation as known in the art. Many basal media formulations (available, e.g., from the American Type Culture Collection, ATCC; or from Invitrogen, Carlsbad, California) can be used to culture the cells herein, including but not limited to Eagle's Minimum Essential Medium (MEM), Dulbecco's Modified Eagle's Medium (DMEM), alpha modified Minimum Essential Medium (alpha-MEM), Basal Medium Essential (BME), BGJb, F-12 Nutrient Mixture (Ham), Iscove's Modified Dulbecco's Medium (IMDM), or X-Vivo-10 serum free medium (clinical grade), available from Invitrogen or Cambrex (New Jersey), and modifications and/or combinations thereof. Compositions of the above basal media are generally known in the art and it is within the skill of one in the art to modify or modulate concentrations of media and/or media supplements as necessary for the cells cultured.

Such basal media formulations contain ingredients necessary for mammal cell development, which are known *per se.* By means of illustration and not limitation, these ingredients may include inorganic salts (in particular salts containing Na, K, Mg, Ca, Cl, P and possibly Cu, Fe, Se and Zn), physiological buffers (e.g., HEPES, bicarbonate), nucleotides, nucleosides and/or nucleic acid bases, ribose, deoxyribose, amino acids, vitamins, antioxidants (e.g., glutathione) and sources of carbon (e.g. glucose, sodium pyruvate, sodium acetate), etc.

For use in culture, basal media can be supplied with one or more further components. For example, additional supplements can be used to supply the cells with the necessary trace elements and substances for optimal growth and expansion. Such supplements include insulin, transferrin, selenium salts, and combinations thereof. These components can be included in a salt solution such as, but not limited to, Hanks' Balanced Salt Solution (HBSS), Earle's Salt Solution. Further antioxidant supplements may be added, e.g., β-mercaptoethanol. While many basal media already contain amino acids, some amino acids may be supplemented later, e.g., L-glutamine, which is known to be less stable when in solution. A medium may be further supplied with antibiotic and/or antimycotic compounds, such as, typically, mixtures of penicillin and streptomycin, and/or other compounds, exemplified but not limited to, amphotericin, ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin, and zeocin.

Lipids and lipid carriers can also be used to supplement cell culture media. Such lipids and carriers can include, but are not limited to cyclodextrin, cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulations.

In embodiments, human plasma or serum may be comprised in said media at a proportion (volume of plasma or serum / volume of medium) between about 0.5% and about 30%, preferably between about 1% and about 15%. The present methods may perform satisfactorily with relatively low amounts of plasma or serum, e.g., about 5 or 10 volume % or below, e.g. about 1, about 2, about 3 or about 4 volume %, allowing to decrease the volume of plasma or serum that needs to be obtained in order to culture the BMSC.

FGF and TGFB are comprised in said media at concentrations sufficient to induce differentiation of BMSC into osteoblasts or osteoblast phenotype cells. Typically, FGF or a biologically active variant or derivative thereof can be included in the media at a concentration of between 0.1 and 100 ng/ml, preferably between 0.5 and 20ng/ml, e.g., at about 19, 18, 17, 16, 15, 14, 13, 12, 11, 10, 9, 8, 7 or 6 ng/ml, or at about 5 ng/ml or less, e.g., at about 4, 3, 2, 1 or 0.5ng/ml. Typically, TGFB or a biologically active variant or derivative thereof can be included in the media at a concentration of between 0.1 and 100 ng/ml, preferably between 0.25 and 20 ng/ml, e.g., at about 19, 18, 17, 16, 15, 14, 13, 12, 11, 10, 9, 8, 7 or 6 ng/ml, or at about 5 ng/ml or less, e.g., at about 4, 3, 2, 1 or 0.5 ng/ml. Said values are intended to refer to concentrations of the respective growth factors or a biologically active variants or derivatives thereof, as exogenously supplemented to the media.

In an embodiment, BMSC or MSC may be continuously contacted with human plasma or serum, FGF and TGFB, i.e., said components would be included in all media in which the BMSC or MSC, the progeny thereof and/or or cells derived there from, are cultured in the present method. Typically, said plasma or serum and growth factors may be supplied at substantially identical respective concentrations in all (fresh) media used for culturing the BMSC or MSC during the method.

In an embodiment, the cells of (a) or (b) may be plated for culturing in step (c) at between 5x10² and 5x10⁶ cells/cm², preferably between 5x10³ and 5x10⁵ cells/cm², more typically at about 5x10⁴ cells/cm².

In a preferred embodiment, the culture vessel may provide for a plastic surface to enable cell adherence. In another embodiment, the surface may be a glass surface. In yet another embodiment, the surface may be coated with an appropriate material conducive to adherence and growth of cells, e.g., Matrigel(R), laminin or collagen.

In embodiments, cells may be allowed to attach for about 1 and 8 days, more typically between about 2 and 6 days, more typically about 4 days before removing the non-adherent matter in step (d).

Typically, the cells can be cultured in steps (c) and (d) taken together for a period of between about 7 and about 18 days, usually between about 11 and about 15 days, and more preferably for about 12-14 days. Otherwise, the cells may be cultured in steps (c) and (d) taken together until their confluence reaches about 60% or more, or about 80% or more, or about 90% or more, or even up to 100%.

In an embodiment, following step (d) the method may comprise collecting the so-obtained cells or cell population.

In another embodiment, following step (d) the acquired cells may be passaged one or more times, typically one or two times, more preferably once. The passage number refers to the number of times that a cell population has been removed from a culture vessel and undergone a subculture, i.e., a passage. For example, passaging may usually include detachment of cells using a bivalent ion chelator (e.g., EDTA or EGTA) and/or trypsin or suitable protease; re-suspending the detached cells; and re-plating the cells in same or a new culture vessel at a desired cell density.

In a preferred embodiment, subsequent to step (d) as above the method further includes step (e) passaging, in particular once, and further culturing the cells or cell population from step (d) in the medium as defined in (c). Following such step (e) the cells or cell population may be collected.

In the passaging step (e), the cells are preferably plated for further culturing at between 5x10¹ and 5x10⁶ cells/cm², preferably between 5x10² and 5x10⁴ cells/cm², more typically at about 5x10³ cells/cm².

Typically, after passaging the cells are cultured in step (e) for a period of between about 3 and about 12 days, usually between about 6 and about 10 days, and more typically for about one week. This period provides for satisfactory expansion of the cells.

The above detailed methods of the invention yield osteoprogenitors, osteoblasts or osteoblast phenotype cells, or populations comprising such, with superior characteristics, such as in particular high expansion rate and low HLA-DR expression, which cells and cell populations are suited for prophylactic or therapeutic implantation in patient bone tissue.

Accordingly, the specification relates to human osteoprogenitors, osteoblasts or osteoblast phenotype cells, or cell populations comprising such, obtainable or directly obtained using the methods of the invention as described above, as well as to such cells or cell populations for use in therapy and/or for use in treating bone-related disorders (e.g., as listed elsewhere in this specification), as well as to the use of such cells or cell populations for the manufacture of a medicament for the treatment of bone-related disorders.

Further, the specification relates to a pharmaceutical composition comprising human osteoprogenitors, osteoblasts or osteoblast phenotype cells, or cell populations comprising such, obtainable or directly obtained by methods of the present invention, and suitable for administration at a site of bone lesion.

The specification also relates to a method for preventing and/or treating bone disease in subjects in need of such treatment, comprising: (i) performing a method of the present invention to obtain human osteoprogenitors, osteoblasts or osteoblast phenotype cells, or cell populations comprising such, as described above, and (ii) administering the so-obtained cells or cell populations to said subject, e.g., at a site of bone lesion, such as for example surgery or fracture.

### 2. Cells and cell populations of the invention

Further study of the osteoblastic cells resulting from the methods allowed to define a new osteoprogenitor, osteoblast or osteoblast phenotype cell type, which may underlie the advantageous properties observed upon use in bone therapy.

In particular, the specification provides human osteoprogenitors, osteoblasts or osteoblast phenotype cells, as well as cell populations comprising such, characterised in that said cells (1) comprise expression of CD90, CD105, CD73, CD63, CD166 and alkaline phosphatase (ALP), more specifically ALP of the bone-liver-kidney type, (2) do not express CD45, CD14, CD19, and (3) less than 25% of the cells, preferably less than 20% of the cells and even more preferably less than 15% of the cells express HLA-DR.

Wherein a cell is said to be positive for a particular marker, this means that a skilled person will conclude the presence or evidence of a distinct signal, e.g., antibody-detectable or detection by reverse transcription polymerase chain reaction, for that marker when carrying out the appropriate measurement, compared to suitable controls. Where the method allows for quantitative assessment of the marker, positive cells may on average generate a signal that is significantly different from the control, e.g., but without limitation, at least 1.5-fold higher than such signal generated by control cells, e.g., at least 2-fold, at least 4-fold, at least 10-fold, at least 20-fold, at least 30-fold, at least 40-fold, at least 50-fold higher or even higher.

The expression of the above cell-specific markers can be detected using any suitable immunological technique known in the art, such as immuno-cytochemistry or affinity adsorption, Western blot analysis, FACS, ELISA, etc., or by any suitable biochemical assay of enzyme activity (e.g., for ALP), or by any suitable technique of measuring the quantity of the marker mRNA, e.g., Northern blot, semi-quantitative or quantitative RT-PCR, etc. Sequence data for markers listed in this disclosure are known and can be obtained from public databases such as GenBank (http://www.ncbi.nlm.nih.gov/).

In a further embodiment, said osteoprogenitor, osteoblast or osteoblast phenotype cells show evidence of ability to mineralize the external surroundings, or synthesize calcium-containing extracellular matrix, when exposed to osteogenic medium (Jaiswal et al. 1997. J Cell Biochem 64: 295-312). Calcium accumulation inside cells and deposition into matrix proteins can be conventionally measured for example by culturing in ⁴⁵Ca²⁺, washing and re-culturing, and then determining any radioactivity present inside the cell or deposited into the extracellular matrix (U.S. Pat. No. 5,972,703), or by assaying culture substrate for mineralization using a Ca²⁺ assay kit (Sigma Kit #587), or as described in the examples.

In another embodiment, said osteoprogenitor, osteoblast or osteoblast phenotype cells substantially do not differentiate towards any one of, and preferably towards neither of cells of adipocytic lineage (e.g., adipocytes) or chondrocytic lineage (e.g., chondrocytes). The absence of differentiation towards these cell lineages may be tested using standard differentiation inducing conditions established in the art (e.g., see Pittenger et al. 1999. Science 284: 143-7), and assaying methods (e.g., when induced, adipocytes typically stain with oil red O showing lipid accumulation; chondrocytes typically stain with alcian blue or safranin O). Substantially lacking propensity towards adipogenic or chondrogenic differentiation may typically mean that less than 65%, or less than 50%, or less than 35%, or less than 20% or less than 10% of the tested cells would show signs of adipogenic or chondrogenic differentiation when applied to the respective test.

In a further embodiment, said osteoprogenitor, osteoblast or osteoblast phenotype cells may also express osteocalcin (OCN) (in particular experiments range from 8 to 85% of the cells expressed OCN as detected by FACS).

In a further embodiment, said osteoprogenitor, osteoblast or osteoblast phenotype cells may also express bone sialoprotein (BSP) (in a particular semi-quantitative RT-PCR experiment the cells expressed weak to moderate levels of BSP).

The human osteoprogenitors, osteoblasts or osteoblast phenotype cells as characterised here above, or populations comprising such, display superior characteristics, such as in particular high expansion rate and low HLA-DR expression, which cells and cell populations are suited for prophylactic or therapeutic implantation in patient bone tissue.

Accordingly, the specification relates to human osteoprogenitors, osteoblasts or osteoblast phenotype cells as characterised here above, or cell populations comprising such, for use in therapy and/or for use in treating bone-related disorders (e.g., as listed elsewhere in this specification), as well as to the use of such cells or cell populations for the manufacture of a medicament for the treatment of bone-related disorders.

Further, the specification relates to a pharmaceutical composition comprising human osteoprogenitors, osteoblasts or osteoblast phenotype cells as characterised here above, or cell populations comprising such, and suitable for administration at a site of bone lesion.

The specification also relates to a method for preventing and/or treating bone disease in subjects in need of such treatment, comprising administering said human osteoprogenitors, osteoblasts or osteoblast phenotype cells as characterised here above, or cell populations comprising such, to said subject, e.g., at a site of bone lesion, such as for example surgery or fracture.

### 3. Aspects relating to cells and populations of the invention

The following description relates to the above stated and further aspects that involve osteoprogenitors, osteoblasts or osteoblast phenotype cells and populations comprising such, as obtained or obtainable by methods described in section 1 above; or osteoprogenitors, osteoblasts or osteoblast phenotype cells, and cell populations comprising such, as characterised in section 2 above.

As explained, the cells or cell populations of the invention may be introduced into the bone of a human subject at the site of surgery or fracture. Introduction of the present osteoblasts to bone is useful in the treatment of bone fractures and bone-related disorders, such as for example those listed elsewhere in this specification.

In an embodiment, the osteoblastic cells may be obtained from BMSC or MSC of a subject into which the differentiated osteoblasts are to be introduced (i.e., autologous cells). In another option, which may be available herein *inter alia* due to the low HLA-DR expression by the present cells, the osteoblastic cells may be obtained from BMSC or MSC of one or more human subjects other that the human subject into which the differentiated osteoblasts are to be introduced (i.e., allogeneic cells). In yet another embodiment, the osteoblastic cells may be obtained from BMSC or MSC of a non-human animal, preferably non-human mammal and introduced to a human subject (i.e., xenogenic cells).

As noted, the osteoblastic cells as described herein may be formulated into and administered as pharmaceutical compositions.

Such pharmaceutical compositions may comprise, in addition to the osteoblastic cells as described herein, a pharmaceutically acceptable excipient, carrier, buffer, preservative, stabiliser, anti-oxidant or other material well known to those skilled in the art. Such materials should be non-toxic and should not interfere with the activity of the cells. The precise nature of the carrier or other material will depend on the route of administration. For example, the composition may be in the form of a parenterally acceptable aqueous solution, which is pyrogen-free and has suitable pH, isotonicity and stability. For general principles in medicinal formulation, the reader is referred to Cell Therapy: Stem Cell Transplantation, Gene Therapy, and Cellular Immunotherapy, by G. Morstyn & W. Sheridan eds., Cambridge University Press, 1996; and Hematopoietic Stem Cell Therapy, E. D. Ball, J. Lister & P. Law, Churchill Livingstone, 2000.

Such pharmaceutical compositions may contain further components ensuring the viability of the cells therein. For example, the compositions may comprise a suitable buffer system (e.g., phosphate or carbonate buffer system) to achieve desirable pH, more usually near neutral pH, and may comprise sufficient salt to ensure isoosmotic conditions for the cells to prevent osmotic stress. For example, suitable solution for these purposes may be phosphate-buffered saline (PBS), sodium chloride solution, Ringer's Injection or Lactated Ringer's Injection, as known in the art. Further, the composition may comprise a carrier protein, e.g., albumin, which may increase the viability of the cells.

The pharmaceutical compositions may comprise further components useful in the repair of bone wounds and defects. For example, such components may include without limitation bone morphogenetic proteins, hydroxyapatite/tricalcium phosphate particles (HA/TCP), gelatine, poly-lactic acid, poly-lactic glycolic acid, hyaluronic acid, chitosan, poly-L-lysine, and collagen. For example, the osteoblastic cells may be combined with demineralised bone matrix (DBM) or other matrices to make the composite osteogenic (bone forming in it own right) as well as osteo-inductive. Similar methods using autologous bone marrow cells with allogeneic DBM have yielded good results (Connolly et al. 1995. Clin Orthop 313:8-18).

The pharmaceutical composition can further include or be co-administered with a complementary bioactive factor such as a bone morphogenetic protein, such as BMP-2, BMP-7 or BMP-4, or any other growth factor. Other potential accompanying components include inorganic sources of calcium or phosphate suitable for assisting bone regeneration (WO 00/07639). If desired, cell preparation can be administered on a carrier matrix or material to provide improved tissue regeneration. For example, the material can be a granular ceramic, or a biopolymer such as gelatine, collagen, osteonectin, fibrinogen, or osteocalcin. Porous matrices can be synthesized according to standard techniques (e.g., Mikos et al., Biomaterials 14:323, 1993; Mikos et al., Polymer 35:1068, 1994; Cook et al., J. Biomed. Mater. Res. 35:513, 1997).

Alternatively or in addition, the BMSC or MSC cells or the resulting osteoblastic cells or cell populations of the invention may be stably or transiently transformed with a nucleic acid of interest prior to introduction into the bone lesion, e.g., a surgery or fracture site, of the subject. Nucleic acid sequences of interest include, but are not limited to those encoding gene products that enhance the growth, differentiation and/or mineralization of osteoblasts. For example, an expression system for BMP-2, can be introduced into the BMSC or MSC in a stable or transient fashion for the purpose of treating non-healing fractures or osteoporosis. Methods of transformation of BMSC or MSC and of osteoblasts are known to those skilled in the art.

Hence, the specification also encompasses methods of producing said pharmaceutical compositions by admixing the cells of the invention with one or more additional components as above.

The specification further relates to an arrangement comprising a surgical instrument for administration of a composition at a site of bone lesion and further comprising the osteoblastic cells or cell populations of the invention, or a pharmaceutical composition comprising said cells or cell populations, wherein the arrangement is adapted for administration of the pharmaceutical composition at the site of bone lesion. For example, a suitable surgical instrument may be capable of injecting a liquid composition comprising cells as described herein at the site of bone lesion.

The osteoprogenitors, osteoblasts or osteoblast phenotype cells or cell populations can be administered in a manner that permits them to graft or migrate to the intended tissue site and reconstitute or regenerate the functionally deficient area. Administration of the composition will depend on the musculoskeletal site being repaired. For example, osteogenesis can be facilitated in concordance with a surgical procedure to remodel tissue or insert a split, or a prosthetic device such as a hip replacement. In other circumstances, invasive surgery will not be required, and the composition can be administered by injection or (e.g., for repair of the vertebral column) using a guidable endoscope.

In an embodiment the pharmaceutical cell preparation as define above may be administered in a form of liquid composition. In embodiments, the cells or pharmaceutical composition comprising such can be administered systemically, topically or at a site of lesion.

In another embodiment, the osteoblastic cells or cell populations may be transferred to and/or cultured on suitable substrate to provide for implants. The substrate on which the cells can be applied and cultured can be a metal, such as titanium, cobalt/chromium alloy or stainless steel, a bioactive surface such as a calcium phosphate, polymer surfaces such as polyethylene, and the like. Although less preferred, siliceous material such as glass ceramics, can also be used as a substrate. Most preferred are metals, such as titanium, and calcium phosphates, even though calcium phosphate is not an indispensable component of the substrate. The substrate may be porous or non-porous.

For example, cells that have proliferated, or that are being differentiated in culture dishes, can be transferred onto three-dimensional solid supports in order to cause them to multiply and/or continue the differentiation process by incubating the solid support in a liquid nutrient medium as described herein, if necessary. Cells can be transferred onto a three-dimensional solid support, e.g. by impregnating said support with a liquid suspension containing said cells. The impregnated supports obtained in this way can be implanted in a human subject. Such impregnated supports can also be re-cultured by immersing them in a liquid culture medium, prior to being finally implanted.

The three-dimensional solid support needs to be biocompatible so as to enable it to be implanted in a human. It can be of any suitable shape such as a cylinder, a sphere, a plate, or a part of arbitrary shape. Of the materials suitable for the biocompatible three-dimensional solid support, particular mention can be made of calcium carbonate, and in particular aragonite, specifically in the form of coral skeleton, porous ceramics based on alumina, on zirconia, on tricalcium phosphate, and/or hydroxyapatite, imitation coral skeleton obtained by hydrothermal exchange enabling calcium carbonate to be transformed into hydroxyapatite, or else apatite-wollastonite glass ceramics, bioactive glass ceramics such as Bioglass(TM) glasses.

The above aspects and embodiments are further supported by the following non-limiting examples.

### EXAMPLES

### Experimental procedures

### Cell culture and plasma preparation

20 to 60ml of heparinized bone marrow (BM) were obtained from iliac crest of patients suffering from bone diseases or from healthy volunteers. BM was mixed with phosphate-buffered saline (PBS, 2v:v) and layered on density gradient Ficoll solution. After centrifugation, mononuclear cells were harvested from the interface and washed twice in PBS. In parallel, serum from patients or healthy donors was obtained after centrifugation of 160 ml of blood drained into dry tubes. The cells were resuspended in DMEM medium supplemented with allogeneic plasma at 15% (autologous plasma may be used with substantially the same results), FGF2 at 10ng/ml and TGFb-1 at 10ng/ml. The cells were plated at 1x10⁸ cells/175cm² flasks and maintained in a 37°C humidified atmosphere containing 5% CO2. The cells were allowed to attach for 1 days prior an initial medium change. Two other partial medium changes (half volume changed) are done at days 7 and 11. Cells were detached at day 14 using trypsin/EDTA solution for 1-5 min at 37°C. The cells were counted and plated at 1x10⁵ cells/175cm² for another week of culture.

### Phenotypic analysis

Intracellular and cell surface markers were analyzed at different time points (day 14 and 21) by flow cytometry. For cell surface markers: cells were incubated with the following conjugated monoclonal antibodies: anti-CD45, CD90, CD105, CD73, CD166, CD63, CD14, CD19, HLA-ABC, HLA-DR, CD28, CD80, CD86, and CTLA-4 for 15 min and then washed with PBS before being centrifuged and re-suspended in 0.3ml PBS. For the intracellular markers (OCN and ALP), a permeabilisation kit was used and the specific anti ALP conjugated monoclonal antibody was used according to the manufacturer protocol (Analis). Cells were then washed with PBS before being re-suspended in 0.3ml PBS.

### ALP assay

Alkaline phosphatase enzymatic activity was measured by a biochemical assay based on the hydrolysis of pNPP (p-Nitrophenyl phosphate). When pNPP is dephosphorylated by ALP, it turns yellow and can be detected by a spectrophotometer at 410nm. The ALP enzymatic activity of the cells is determined on day 21 with respect to a standard curve based on purified calf intestinal alkaline phosphatase activity. The ALP activity is reported in Unit of ALP/mg of protein. One unit of ALP hydrolyzes 1µmol of pNPP in 1 min at 37°C.

### Mineralization assay

Induction of cellular mineralization potential was assessed by adding osteogenic medium in the culture. At day 14, 5,700 bone-forming cells/cm² were plated into 6-well plates in the presence of plasma supplemented with 0.1 µM dexamethasone, 0.05 mM ascorbic acid and 3 mM glycerol phosphate. After 2 weeks of culture, cells were fixed in 3.7% formaldehyde/PBS and stained by alizarin red. A mineralization score was given to each culture as follow: 0= no mineralization, 1= ongoing mineralization and 2 = complete mineralization.

### T cell proliferation assay

2,000 to 20,000 pre-osteoblastic cells/ml from individual A (PREOBa) supplemented or not with 10 ng/ml TGFb-1 were co-cultured with 200,000 T cells/ml from individual B (PBMCb). Phytohemaglutinine (PHA) at a concentration of 10µg/ml was used as positive control of T cells proliferation. The cells were cultured in 24-well microtiter plate for 7 days and pulsed with 3H-thymidine for the last 18h of the culture period to measure T cell proliferation. Cells were washed twice with ice-cold PBS and twice with Ice-cold 5% trichloroacetic acid (TCA). Finally, cells were lysed by a solution containing 0.1N NaOH and 0.1% Triton-X100. The supernatant is harvested and mixed with scintillation liquid to be analysed on a beta-counter.

### Results

### Plasma and TGF vs. Plasma and TGF/FGF2

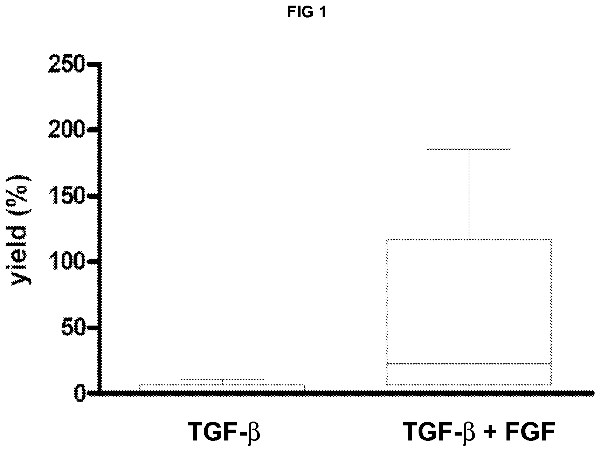

We first tested the effect of TGFb-1 as sole growth factor added to 15% allogeneic plasma containing medium. As is shown in Figure 1, the primary culture with only TGFB-1 failed to give an appropriate amount of cells to be expanded: indeed, there is nearly 20 fold more cells harvested from medium containing both FGF2 and TGFb-1 flasks than in flasks containing TGFb-1 alone (p = 0.0317, two-tailed Mann Whitney test).

### Plasma and FGF vs. plasma and TGF/FGF2

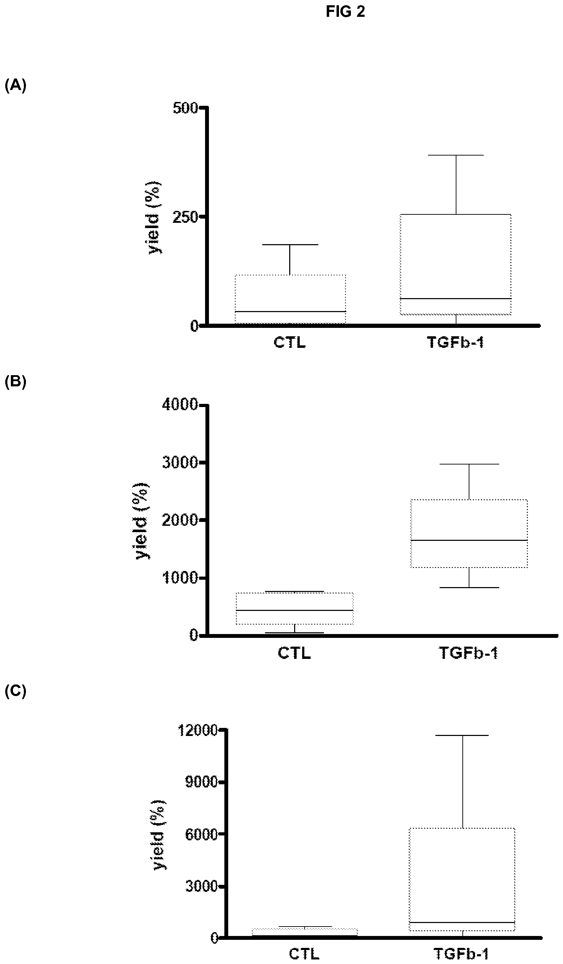

The proliferation rate of osteoblasts from 15 patients in medium containing allogeneic plasma and FGF2 supplemented or not with 10 ng/ml TGFb-1 was assessed (Figure 2). The following experiments showed that the mean yield of expansion of cells cultured in plasma supplemented with TGFb-1 is largely superior to the yield of cells cultured without TGFb-1 both in primary (1.07x), secondary (2.5x) and global (3.08x) cultures (Table 1).

**Table 1: Culture yields of cells cultured with FGF2 and FGF2/TGFb-1**

| | 15% plasma FGF2 | | | 15% plasma FGF2/TGFb-1 | | |
|---|---|---|---|---|---|---|
| % vs # cells plated | Primary culture yield (P1) | Secondary culture yield (P2) | Global yield | Primary culture yield (P1) | Secondary culture yield (P2) | Global yield |
| Mean | 51.3 | 625.3 | 297.4 | 54.4 | 1553.7 | 914.8 |
| SD | 44.5 | 322.7 | 283.1 | 71.3 | 798.1 | 1343.8 |

Moreover, the expansion rate of cells cultured with TGFb-1 was maintained over a longer time period with respect to cells cultured in the absence of TGF as show in Table 2. Cells cultured only with FGF stopped to proliferate after approximately 4 passages while cells cultured in the presence of both FGF2 and TGFb-1 continue to proliferate up to 7 passages. This translates in an increase in proliferation of over 800 times (857.1).

**Table 2: Cumulative yields of cells cultured with FGF2 and FGF2/TGFb-1 (%)**

| % | **FGF** | **FGF/TGF** |
|---|---|---|
| P1 | 51,3 | 54,5 |
| P2 | 32.213,3 | 84.649,4 |
| P3 | 103.144,8 | 1.144.807,1 |
| P4 | **177.156,9** | 9.370.159,7 |
| P5 | 131.512,9 | 31.860.445,3 |
| P6 | 72.555,5 | 78.055.251,8 |
| P7 | 28.637,9 | **151.841.324,1** |
| P8 | 12.554,9 | 120.405.153,2 |
| P9 | 6.364,8 | 71.159.511,4 |
| P10 | 1.912,5 | 24.220.683,9 |
| P11 | 2.616,0 | 9.910.647,4 |
| P12 | 1.620,1 | 254.1373,2 |
| P13 | 447,5 | 112.5860,9 |
| P14 | 311,1 | 123.3310,1 |
| P15 | 28,3 | 587.957,5 |

### Cell phenotype

Flow cytometry revealed that the phenotype of cells obtained in medium supplemented with both FGF2 and TGFb-1 was similar to that of cells cultured without TGFb-1 for most markers. Both cell types expressed CD90, CD105, CD73, CD63 CD166 and ALP but no expression of CD45, CD14 or CD19 was observed (Table 3). Expression ALP in FGF2/TGFb-1 culture conditions was reduced as compared to FGF2 sole conditions (42.2% vs. 73.6% respectively).

Moreover, cells incubated with TGFb-1 expressed high levels of HLA-ABC (HLA-l) but low levels of HLA-DR in comparison with cells cultured without this factor. Furthermore, all the co-stimulatory molecules tested (CD80, CD86, CD28, CD40L and CTLA-4) were expressed at negligible levels in TGFb-1 treated cells (Table 3).

**Table 3: Phenotypic markers of osteoblasts cultured in medium supplemented or not supplemented with 10 ng/ml TGFb-1 (percentage of cells deemed positive by FACS).**

| | 15% Plasma/FGF2 | | | 15% Plasma/FGF2/TGFb-1 | | |
|---|---|---|---|---|---|---|
| | N | Mean | SD | N | Mean | SD |
| ALP | 34 | 73,57 | 17,55 | 22 | 42,18 | 24,82 |
| CD105 | 34 | 90,68 | 8,03 | 24 | 93,08 | 5,71 |
| CD90 | 9 | 96,33 | 4,22 | 4 | 94,75 | 2,95 |
| CD73 | 10 | 97,20 | 1,60 | 6 | 97,50 | 1,71 |
| CD166 | 9 | 92,56 | 5,21 | 4 | 94,00 | 4,85 |
| CD63 | 9 | 71,11 | 20,73 | 5 | 68,00 | 25,26 |
| | | | | | | |
| CD45 | 33 | 2,29 | 2,43 | 24 | 1,92 | 1,00 |
| CD14 | 16 | 1,69 | 1,61 | 12 | 1,42 | 1,11 |
| CD19 | 16 | 9,38 | 11,93 | 9 | 4,00 | 2,62 |
| | | | | | | |
| HLA-DR | 10 | 62,90 | 19,26 | 22 | 6,14 | 5,80 |
| HLA-l | 9 | 94,78 | 5,22 | 22 | 95,18 | 3,71 |
| B7-2 | 9 | 3,67 | 2,71 | 6 | 1,17 | 1,07 |
| CD28 | 9 | 2,00 | 1,94 | 6 | 0,83 | 0,37 |
| CD80 | 9 | 2,22 | 1,03 | 11 | 0,91 | 0,67 |
| CD40L | 9 | 1,89 | 1,66 | 4 | 1,00 | 0,71 |
| CTLA4 | 9 | 6,11 | 3,66 | 4 | 5,00 | 4,74 |

### ALP enzymatic activity:

The ALP enzymatic activity of the cells was assessed by a colorimetric assay and show that cells cultured with TGFb-1 have a lower ALP activity with respect to cells cultured without this growth factor (Table 4). These results strongly correlate with the ALP expression measured by FACS.

**Table 4: ALP activity, ALP expression and mean intensity of fluorescence (MIF) for cells cultured in FGF2 and FGF2/TGFb-1 at passage 2**

| | 15% Plasma FGF | | | 15% Plasma FGF/TGF | | |
|---|---|---|---|---|---|---|
| | ALP activity (mU/mg) | Cellular expression (%) | MIF | ALP activity (mU/mg) | Cellular expression (%) | MIF |
| Mean | 1129.02 | 71.78 | 85.85 | 281.00 | 55.63 | 19.94 |
| SD | 1196.69 | 18.68 | 47.59 | 320.08 | 22.43 | 9.50 |

### Mineralization:

Even with relatively lower ALP expression, cells cultured with FGF2/TGFb-1 were shown to display the same biological activity (mineralization) as the cells cultured with FGF2 only. This was determined by mineralization.

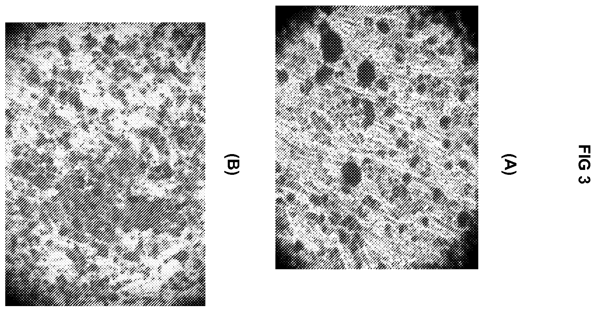

Mineralization of cells cultured in presence of FGF2 (A) or FGF2/TGFb-1 (B) was identical (over 65% of the well surface mineralized - Figure 3). Similarly, the mean of the mineralization score of cells cultured in FGF2 (1,75) overlapped the score of cells cultured in FGF2/TGFb-1 (1.83), out of a maximum of 2.

### T cell proliferation

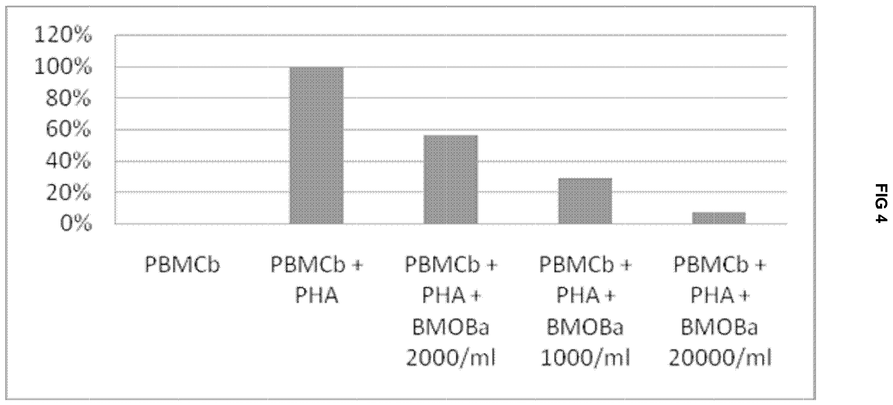

To determine whether bone forming cells cultured in presence of TGFb-1 induce a proliferative response by allogeneic T cells, peripheral blood mononucleated cells (PBMC) were cultured with increasing numbers of bone forming cells (2,000-20,000/ml). As shown in Table 5 and Figure 4, FGF2/TGFb-1 produced osteoblasts did not induce an allogeneic response of PBMC (left side) and were also able to induce up to 90% inhibition of proliferation of PHA stimulated PBMC.

**Table 5 Inhibitory effects of osteoblasts on T cells proliferation (value are presented as percentages of PHA/PBMC proliferation).**

| | **FGF + TGF** | | | | |
|---|---|---|---|---|---|
| | PBMCb | PBMCb + PHA | PBMCb + PHA + BMOBa (2000/ml) | PBMCb + PHA + BMOBa (10000/ml) | PBMCb + PHA + BMOBa (20000/ml) |
| 1 | 0 | 100 | 61 | 41 | 18 |
| 2 | 0 | 100 | 48 | 29 | 8 |
| 3 | 0 | 100 | 56 | 29 | 8 |
| 4 | 0 | 100 | 62 | 56 | 17 |
| 5 | 0 | 100 | 61 | 41 | 18 |

### Conclusions

In order to develop a method to rapidly and significantly expand immunoprivileged BMSC or MSC derived bone-forming cells, the potency of TGFB has been assessed on those cell growth and characteristics.

Surprisingly, culture condition with TGFb and plasma alone was not able to expand and differentiate MSC into bone forming cells while the combination with FGF2 has a very significant expansion and differentiation effect (expansion of up to 12 fold of serum/FGF alone). Moreover, the phenotypic status of the cells cultured in presence of TGFb remained unchanged for stromal and haematopoietic markers, only ALP expression was slightly diminished although this had no effect on the cells biological activities. Interestingly, HLA-DR expression in the TGFb conditions was present at very low/undetectable level on the bone cells. In addition, in the mixed lymphocyte reaction that cells cultured in presence of TGFb in the plasma/FGF conditions are immunoprivileged as they do not induce an allogeneic reaction and therefore would not lead to an immune rejection in case of allogeneic transplantation. Finally we have shown that cells growing in presence of TGFb keep their biological functions.

We can conclude that the use of medium supplemented with TGFb is superior to medium conditions without TGFb to expand BMSC or MSC into osteoblastic cells without altering cell phenotypes.

In further examples, the above described experimental procedures are performed in a substantially same or similar manner using a FGF factor other than FGF2 (e.g., FGF-1 or FGF-3) and/or using TGF factor other than TGFb-1 (*e.g*., TGFb-2 or TGFb-3), giving comparable results and/or effects.

## Claims

1. A method for obtaining osteoprogenitors or osteoblasts from adult human bone marrow stem cells (BMSC) or adult human mesenchymal stem cells (MSC) *in vitro* or *ex vivo,* comprising culturing said BMSC or MSC in a medium including human plasma or serum, fibroblast growth factor (FGF) and transforming growth factor beta (TGFB).

2. The method according to claim 1, comprising steps:
(a) recovering cells from a biological sample of a human subject comprising BMSC or MSC;
(b) optionally, isolating mono-nucleated cells from the cells recovered in (a);
(c) adding cells of (a) or (b) to a medium comprising human plasma or serum, FGF and TGFB and culturing the cell-medium mixture, such as to allow for adherence of cells to a substrate surface;
(d) removing non-adherent matter and further culturing adherent cells in the medium as defined in (c), such as to allow for obtaining osteoprogenitors or osteoblasts.

3. The method according to claim 2, wherein the cells are cultured in steps (c) and (d) taken together for a period of between about 7 and about 18 days.

4. The method according to any of claims 2 or 3 further comprising collecting the cells obtained in step (d).

5. The method according to any of claims 2 or 3 further including step (e) passaging and further culturing the cells from step (d) in the medium as defined in (c).

6. The method according to claim 5, wherein the cells are cultured in step (e) for a period of between about 3 and about 12 days.

7. The method according to any of claims 1 to 6, wherein the FGF is FGF-1, FGF-2 or FGF-3.

8. The method according to any of claims 1 to 7, wherein the TGFB is TGFB-1, TGFB-2 or TGFB-3.

9. A cell population comprising human osteoprogenitors or osteoblasts, **characterised in that** said osteoprogenitors or osteoblasts (1) comprise expression of CD90, CD105, CD73, CD63, CD166, alkaline phosphatase (ALP), more specifically ALP of the bone-liver-kidney type, (2) do not express CD45, CD14, CD19, and (3) less than 15% of the cells express HLA-DR.

10. The cell population according to claim 9, obtainable by the method of any of claims 1 to 8.

11. The cell population according to claims 9 or 10, wherein said osteoprogenitors or osteoblasts further comprise expression of osteocalcin (OCN).

12. The cell population as defined in any of claims 9 to 11 for use in treating a bone-related disorder.

13. The cell population for use according to claim 12, wherein the cell population is to be administered to an allogeneic subject.

14. A pharmaceutical composition comprising the cell population as defined in any of claims 9 to 11.

15. The pharmaceutical composition according to claim 14, wherein the pharmaceutical composition is suitable for administration of said cell population at a site of bone lesion.

16. An arrangement comprising a surgical instrument for administration of a composition at a site of bone lesion and further comprising the pharmaceutical composition as defined in claim 14 or 15, wherein the arrangement is adapted for administration of the pharmaceutical composition at the site of bone lesion.

## Patentansprüche

1. Verfahren zur Gewinnung von Osteoprogenitoren oder Osteoblasten aus adulten menschlichen Knochenmarkstammzellen (BMSC) oder adulten menschlichen mesenchymalen Stammzellen (MSC) *in vitro* oder *ex vivo,* umfassend das Kultivieren der BMSC oder MSC in einem Medium, das menschliches Plasma oder Serum, Fibroblastenwachstumsfaktor (FGF) und transformierenden Wachstumsfaktor Beta (TGFB) enthält.

2. Verfahren nach Anspruch 1, umfassend die Schritte:
(a) Rückgewinnung von Zellen aus einer biologischen Probe von einem Menschen, umfassend BMSC oder MSC;
(b) optional Isolieren mononukleärer Zellen aus den in (a) rückgewonnenen Zellen;
(c) Zugeben von Zellen aus (a) oder (b) zu einem Medium, das menschliches Plasma oder Serum, FGF und TGFB umfasst, sowie Kultivieren des Zellen-Medium-Gemischs derart, dass das Anhaften von Zellen an einer Substratoberfläche ermöglicht wird;
(d) Entfernen nicht adhärenter Materie und Weiterkultivieren adhärenter Zellen in dem unter (c) definierten Medium derart, dass die Gewinnung von Osteoprogenitoren oder Osteoblasten ermöglicht wird.

3. Verfahren nach Anspruch 2, wobei die Zellen in den Schritten (c) und (d) zusammengenommen für eine Dauer von zwischen ungefähr 7 und ungefähr 18 Tagen kultiviert werden.

4. Verfahren nach einem der Ansprüche 2 oder 3, ferner das Sammeln der in Schritt (d) gewonnenen Zellen umfassend.

5. Verfahren nach einem der Ansprüche 2 oder 3, ferner Schritt (e) Passagieren und Weiterkultivieren der Zellen aus Schritt (d) in dem unter (c) definierten Medium umfassend.

6. Verfahren nach Anspruch 5, wobei die Zellen in Schritt (e) für eine Dauer von zwischen ungefähr 3 und ungefähr 12 Tagen kultiviert werden.

7. Verfahren nach einem der Ansprüche 1 bis 6, wobei es sich bei dem FGF um FGF-1, FGF-2 oder FGF-3 handelt.

8. Verfahren nach einem der Ansprüche 1 bis 7, wobei es sich bei dem TGFB um TGFB-1, TGFB-2 oder TGFB-3 handelt.

9. Eine menschliche Osteoprogenitoren oder Osteoblasten umfassende Zellpopulation, **dadurch gekennzeichnet, dass** die Osteoprogenitoren oder Osteoblasten (1) Expression von CD90, CD105, CD73, CD63, CD166, alkalische Phosphatase (ALP), insbesondere ALP des Knochen-Leber-Nieren-Typs, umfassen, (2) nicht CD45, CD14, CD19 exprimieren und (3) weniger als 15 % der Zellen HLA-DR exprimieren.

10. Zellpopulation nach Anspruch 9, erhältlich durch dem Verfahren einer der Ansprüche 1 bis 8.

11. Zellpopulation nach den Ansprüchen 9 oder 10, wobei die Osteoprogenitoren oder Osteoblasten ferner die Expression von Osteocalcin (OCN) umfassen.

12. Zellpopulation gemäß der Definition in einem der Ansprüche 9 bis 11 zur Anwendung bei der Behandlung einer Knochenerkrankung.

13. Zellpopulation zur Anwendung nach Anspruch 12, wobei die Zellpopulation einem allogenen Patienten zu verabreichen ist.

14. Pharmazeutische Zusammensetzung, umfassend die in einem der Ansprüche 9 bis 11 definierte Zellpopulation.

15. Pharmazeutische Zusammensetzung nach Anspruch 14, wobei die pharmazeutische Zusammensetzung zur Anwendung der Zellpopulation am Ort einer Knochenläsion geeignet ist.

16. Anordnung, umfassend ein chirurgisches Instrument zur Anwendung einer Zusammensetzung am Ort einer Knochenläsion und ferner umfassend die in Anspruch 14 oder 15 definierte pharmazeutische Zusammensetzung, wobei die Anordnung zur Anwendung der pharmazeutischen Zusammensetzung am Ort der Knochenläsion ausgelegt ist.

## Revendications

1. Procédé d'obtention d'ostéoprogéniteurs ou d'ostéoblastes à partir de cellules souches de moelle osseuse (BMSC) d'humain adulte ou de cellules souches mésenchymateuses (MSC) d'humain adulte *in vitro* ou *ex vivo,* comprenant la culture desdites BMSC ou MSC dans un milieu comprenant du plasma ou sérum humain, du facteur de croissance des fibroblastes (FGF) et du facteur de croissance transformant bêta (TGFB).

2. Procédé selon la revendication 1, comprenant les étapes de :
(a) récupération de cellules à partir d'un échantillon biologique d'un sujet humain comprenant des BMSC ou des MSC ;
(b) facultativement, isolement de cellules mononucléaires à partir des cellules récupérées dans (a) ;
(c) ajout des cellules de (a) ou (b) à un milieu comprenant du plasma ou du sérum humain, FGF et TGFB et culture du mélange cellules-milieu, de manière à permettre l'adhésion de cellules à une surface de substrat ;
(d) élimination de la matière non adhérente et ensuite, culture des cellules adhérentes dans le milieu tel que défini dans (c), de manière à permettre l'obtention d'ostéoprogéniteurs ou d'ostéoblastes.

3. Procédé selon la revendication 2, dans lequel les cellules sont cultivées dans les étapes (c) et (d) pendant une durée totale comprise entre environ 7 et environ 18 jours.

4. Procédé selon l'une quelconque des revendications 2 ou 3 comprenant en outre la collecte des cellules obtenues dans l'étape (d).

5. Procédé selon l'une quelconque des revendications 2 ou 3 comprenant en outre l'étape (e) de passage et ensuite culture des cellules de l'étape (d) dans le milieu tel que défini dans (c).

6. Procédé selon la revendication 5, dans lequel les cellules sont cultivées dans l'étape (e) pendant une durée comprise entre environ 3 et environ 12 jours.

7. Procédé selon l'une quelconque des revendications 1 à 6, dans lequel le FGF est FGF-1, FGF-2 ou FGF-3.

8. Procédé selon l'une quelconque des revendications 1 à 7, dans lequel le TGFB est TGFB-1, TGFB-2 ou TGFB-3.

9. Population de cellules comprenant des ostéoprogéniteurs ou ostéoblastes humains, **caractérisée en ce que** lesdits ostéoprogéniteurs ou ostéoblastes (1) comprennent l'expression de CD90, CD105, CD73, CD63, CD166, phosphatase alcaline (ALP), plus spécifiquement ALP du type os-foie-rein, (2) n'expriment pas CD45, CD14, CD19, et (3) moins de 15 % des cellules expriment HLA-DR.

10. Population de cellules selon la revendication 9, pouvant être obtenue par le procédé de l'une quelconque des revendications 1 à 8.

11. Population de cellules selon les revendications 9 ou 10, dans laquelle lesdits ostéoprogéniteurs ou ostéoblastes comprennent en outre l'expression d'ostéocalcine (OCN).

12. Population de cellules telle que définie dans l'une quelconque des revendications 9 à 11 pour utilisation dans le traitement d'un trouble osseux.

13. Population de cellules pour utilisation selon la revendication 12, la population de cellules étant destinée à être administrée à un sujet allogénique.

14. Composition pharmaceutique comprenant la population de cellules telle que définie dans l'une quelconque des revendications 9 à 11.

15. Composition pharmaceutique selon la revendication 14, la composition pharmaceutique étant adaptée pour administration de ladite population de cellules à un site de lésion osseuse.

16. Agencement comprenant un instrument chirurgical pour administration d'une composition à un site de lésion osseuse et comprenant en outre la composition pharmaceutique telle que définie dans la revendication 14 ou 15, l'agencement étant adapté pour administration de la composition pharmaceutique au site de lésion osseuse.
